# EUROPEAN PATENT APPLICATION

(11) **EP 4 029 453 A1**
(43) Date of publication of application: **20.07.2022**
(21) Application number: 21151255.3
(22) Date of filing: 13.01.2021
(51) Int. Cl.: A61B 8/02, A61B 8/08, A61B 8/00

(54) **AN APPARATUS FOR MONITORING A HEARTBEAT OF A FETUS**

(71) Applicant: Koninklijke Philips N.V., 5656 AG Eindhoven (NL)
(72) Inventor: VAJINEPALLI, Pallavi, 5656 AE Eindhoven (NL); RADHAKRISHNAN, Ravindranath, 5656 AE Eindhoven (NL); GEYWITZ, Hansjoerg, 5656 AE Eindhoven (NL); KRISHNAN, Karthik, 5656 AE Eindhoven (NL); KAUSHAL, Nitesh, 5656 AE Eindhoven (NL); FIRTION, Celine, 5656 AE Eindhoven (NL); PHAM, Hoa, 5656 AE Eindhoven (NL)
(74) Representative: Philips Intellectual Property & Standards

(57) **Abstract**

An apparatus for monitoring a heartbeat of a fetus comprises a memory comprising instruction data representing a set of instructions, and a processor configured to communicate with the memory and to execute the set of instructions. The set of instructions, when executed by the processor, cause the processor to: i) obtain ultrasound image data from an ultrasound transducer, ii) determine a location of the heart of the fetus in the ultrasound image data, iii) send a message to the ultrasound transducer to instruct the ultrasound transducer to switch to a Doppler mode, wherein the determined location of the heart of the fetus is used to set the target location of the Doppler mode, and iv) monitor the heartbeat of the fetus using ultrasound data obtained in the Doppler mode.

## Description

### FIELD OF THE INVENTION

The disclosure herein relates to ultrasound systems. Particularly but non-exclusively, the disclosure relates to ultrasound systems, apparatus and methods for monitoring a heartbeat of a fetus.

### BACKGROUND OF THE INVENTION

During labour and delivery, some of the most common obstetric procedures include fetal heart rate (FHR) monitoring and the monitoring of maternal contractions. These measures enable healthcare workers to assess the state of the fetus and to determine if intervention is required. Optimal FHR monitoring requires a collaborative perinatal team that performs the monitoring correctly, interprets it appropriately, and communicates the findings effectively, and in a timely fashion, to all members of the care team when a high-risk pattern is detected. Hence appropriate FHR acquisition and monitoring plays a big role in FHR measurement.

Fetal heart rate monitors generally comprise a transducer that is placed on the mother's abdomen and held in place with a strap or belt. The placement of the transducer is still generally performed using the acoustic feedback that results from the Doppler shift. A correct and optimal placement of the transducer is essential as there may be other sources of Doppler shift in the detection range of the ultrasonic transducer. For example, blood flow in large maternal blood vessels is a major cause of mismeasurements. The heart rate calculation can also abruptly change from the fetal heart rate to the maternal heart rate, depending on which signal is more strongly received. The superposition of the two signals, which occurs quite frequently, can also lead to a gradual change in the heart rate values, which may not be recognized at first glance. Furthermore, the ultrasound sensor can lose its focus on the fetal heart due to the movement of the fetus or a movement of the mother.

Thus FHR measurement in many current fetal monitors is reliant on the clinical expertise of the health care professional in placing and maintaining the fetal monitor transducer over the fetal heart. High levels of training are therefore generally required to both optimally place the transducer and to maintain the transducer at the correct position (as the fetus/mother moves). Thus in some situations, training and understanding of the clinician (e.g. operating personnel) may not be sufficient to place the transducer optimally.

### SUMMARY OF THE INVENTION

As described above, ultrasound transducers for use in fetal monitoring during labour can be hard to place correctly in order to monitor the fetal heartbeat. Furthermore, they require constant monitoring to maintain correct placement as the fetus and/or mother moves. A frequent problem with the use of such monitors is that the heart rate obtained is from the mother instead of the fetus. Such issues are further complicated in pregnancies with multiple fetuses, where, due to the movement of the fetuses, over time, a fetal heart rate monitor may begin to monitor the wrong fetus. It is also difficult to ensure that fetal heart rate measurements are consistently attributed to the correct fetus in the event of multiples.

Furthermore, placing the transducer using only the Doppler shift as feedback requires a real-time transmission of the sound. In case of a wireless system, this limits the selection of the radio technology because modern protocols often have delays of some 100ms which is not acceptable when placing the transducer on an acoustic feedback basis. It is an object of the disclosure herein to provide improved fetal monitoring systems to address some of these issues.

According to a first aspect, there is provided an apparatus for monitoring a heartbeat of a first fetus. The apparatus comprises a memory comprising instruction data representing a set of instructions and a processor configured to communicate with the memory and to execute the set of instructions. The set of instructions, when executed by the processor, cause the processor to: i) obtain ultrasound image data from an ultrasound transducer; ii) determine a location of the heart of the first fetus in the ultrasound image data; iii) send a message to the ultrasound transducer to instruct the ultrasound transducer to switch to a Doppler mode, wherein the determined location of the heart of the first fetus is used to set the target location of the Doppler mode; and iv) monitor the heartbeat of the first fetus using ultrasound data obtained in the Doppler mode.

According to a second aspect there is provided an ultrasound system comprising the apparatus of the first aspect and further comprising the ultrasound transducer.

According to a third aspect there is a computer implemented method of monitoring a heartbeat of a first fetus. The method comprises i) obtaining ultrasound image data from an ultrasound transducer; ii) determining a location of the heart of the first fetus in the ultrasound image data; iii) sending a message to the ultrasound transducer to instruct the ultrasound transducer to switch to a Doppler mode, wherein the determined location is used to set the target location of the Doppler mode; and iv) monitoring the heartbeat of the first fetus using ultrasound data obtained in the Doppler mode.

According to a fourth aspect there is a computer program product comprising a computer readable medium, the computer readable medium having computer readable code embodied therein, the computer readable code being configured such that, on execution by a suitable computer or processor, the computer or processor is caused to perform the method of the third aspect.

Thus the apparatus, systems and methods herein allow for "soundless" placement of ultrasound transducers on fetuses, whereby an imaging mode is used for the correct placement of the transducer, rather than relying solely on acoustic feedback. A "soundless" placement with indicators where to move the transducer to could also be advantageous in areas where multiple women are monitored in one room. Furthermore, the systems and methods herein enable the fetus to be monitored even if the fetus changes position. It also gives certainty that the fetal heart is being monitored (e.g. as opposed to the mother's heart). In embodiments (as described below) where two or more fetuses are monitored, the methods and apparatuses herein give certainty that each fetus is correctly monitored, e.g. without any confusion/overlap between the different fetuses' FHRs.

These and other aspects will be apparent from and elucidated with reference to the embodiment(s) described hereinafter.

### BRIEF DESCRIPTION OF THE DRAWINGS

Example embodiments will now be described, by way of example only, with reference to the following drawings, in which:
Fig. 1 shows an apparatus according to some embodiments herein;
Fig. 2 shows an ultrasound system according to some embodiments herein;
Fig. 3 shows an example apparatus according to some embodiments herein;
Fig. 4 shows an example output of a YOLO network according to some embodiments herein;
Fig. 5 shows Doppler gating;
Fig. 6 shows an example process according to some embodiments herein;
Fig. 7 shows another example process according to some embodiments herein;
Fig. 8 shows an example output of a YOLO network on an image comprising two fetal hearts;
Fig. 9a and Fig 9b show example landmarks on a fetal heart; and
Fig 10 shows an example method according to some embodiments herein.

### DETAILED DESCRIPTION OF EMBODIMENTS

As described above, fetal heart rate monitors whereby a fetal heart-rate is detected and monitored using the acoustic feedback that results from the Doppler shift are difficult to correctly place and difficult to maintain in the correct position as the fetus and/or mother moves. These issues are compounded for pregnancies involving multiples where there is more than one fetus present. It is an object of embodiments herein to improve on this situation to provide improved fetal heart rate monitoring during labour.

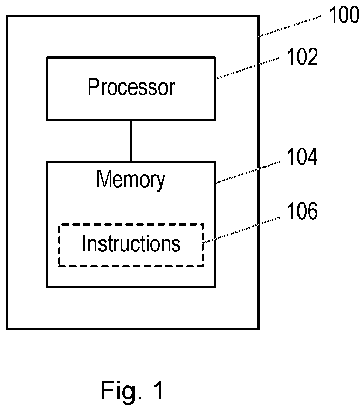

Turning to Fig. 1, which shows an apparatus 100 for monitoring a heartbeat of a first fetus, according to some embodiments herein. Generally, the apparatus may form part of a computer apparatus or system e.g. such as a laptop, desktop computer or other computing device. In some embodiments, the apparatus 100 may form part of a distributed computing arrangement or the cloud.

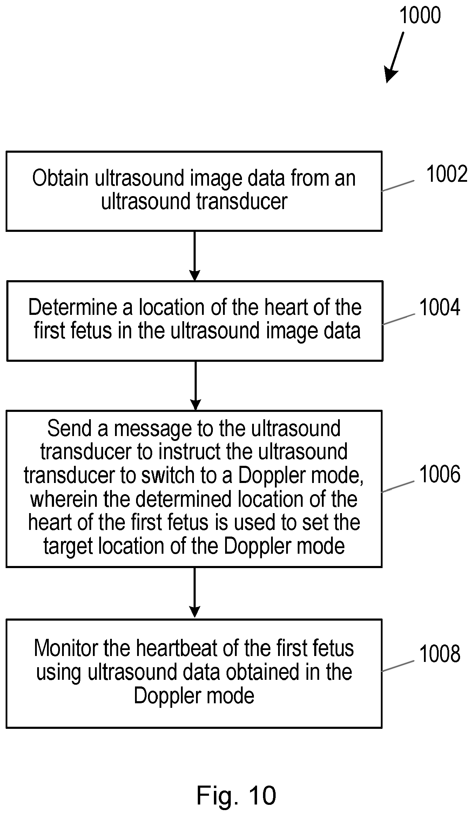

Generally, the apparatus may be adapted or configured to perform any of the embodiments of the methods described herein, such as the method 1000 as described below with respect to Fig. 10.

The apparatus comprises a memory 104 comprising instruction data representing a set of instructions 106 and a processor 102 (e.g. processing circuitry or logic) configured to communicate with the memory and to execute the set of instructions 106. Briefly, the set of instructions 106, when executed by the processor 102, cause the processor 102 i) obtain ultrasound image data from an ultrasound transducer; ii) determine a location of the heart of the first fetus in the ultrasound image data; iii) send a message to the ultrasound transducers to instruct the ultrasound transducer to switch to a Doppler mode, wherein the determined location of the heart of the first fetus is used to set the target location of the Doppler mode; and iv) monitor the heartbeat of the first fetus using ultrasound data obtained in the Doppler mode.

As described above, a major problem with current ultrasound transducers used in obstetric fetal monitoring is that they are difficult to correctly place and difficult to maintain in the correct position, in order to monitor the fetal heart. Movement of the fetus or mother, ambient noise, the maternal heart-beat and other sounds, e.g. from blood moving through maternal veins and arteries may all lead to inaccurate placement and monitoring of the fetal heart. The proposed apparatus herein uses an imaging mode of the transducer in order to determine the location of the fetal heart. The determined location is then used to set the target location of a Doppler mode (e.g. it may be used to set a Doppler gate) to monitor the fetal heartbeat, ensuring that it is the fetal heart that is monitored, e.g. as opposed to any of the other aforementioned motions. This therefore provides more robust fetal monitoring.

In more detail, the processor 102 may comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method described herein. The processor 102 can comprise one or more processors, processing units, multi-core processors and/or modules that are configured or programmed to control the apparatus 100 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store program code that can be executed by the processor 102 to perform the method described herein. Alternatively or in addition, one or more memories 104 may be external to (i.e. separate to or remote from) the apparatus 100. For example, one or more memories 104 may be part of another device. Memory 104 can be used to store the obtained ultrasound data and/or any other information or data received, calculated or determined by the processor 102 of the apparatus 100 or from any interfaces, memories or devices that are external to the apparatus 100. The processor 102 may be configured to control the memory 104 to store the obtained ultrasound data and/or the any other information or data received, calculated or determined by the processor.

In some embodiments, the memory 104 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 1 only shows the components required to illustrate this aspect of the disclosure and, in a practical implementation, the apparatus 100 may comprise additional components to those shown. For example, the apparatus 100 may further comprise a display. A display may comprise, for example, a computer screen, and/or a screen on a mobile phone or tablet. The apparatus may further comprise a user input device, such as a keyboard, mouse or other input device that enables a user to interact with the apparatus, for example, to provide initial input parameters to be used in the method described herein. The apparatus 100 may comprise a battery or other power supply for powering the apparatus 100 or means for connecting the apparatus 100 to a mains power supply.

In some embodiments, the apparatus 100 may be comprised in an ultrasound system. For example, an ultrasound system may comprise the apparatus 100 described above, and further comprise a transducer with which to record the sequence of ultrasound image data.

In some embodiments, an ultrasound system may further comprise a display. The processor may be caused to send an instruction to the display to display the ultrasound image data and/or the ultrasound data obtained in the Doppler mode on the display. The processor may further be caused to send an instruction to the display to cause the display to provide an indication of whether the ultrasound transducer is operating in an imaging mode or a Doppler mode.

In some embodiments as described below, an ultrasound system may comprise an array of transducers, arranged into a wearable "patch". The patch may comprise drive electronics to activate each transducer in the array individually. The transducers may further be fitted with sensors (such as, for example, Inertial Measurement Unit, IMU sensors) to determine the orientation of their respective transducers.

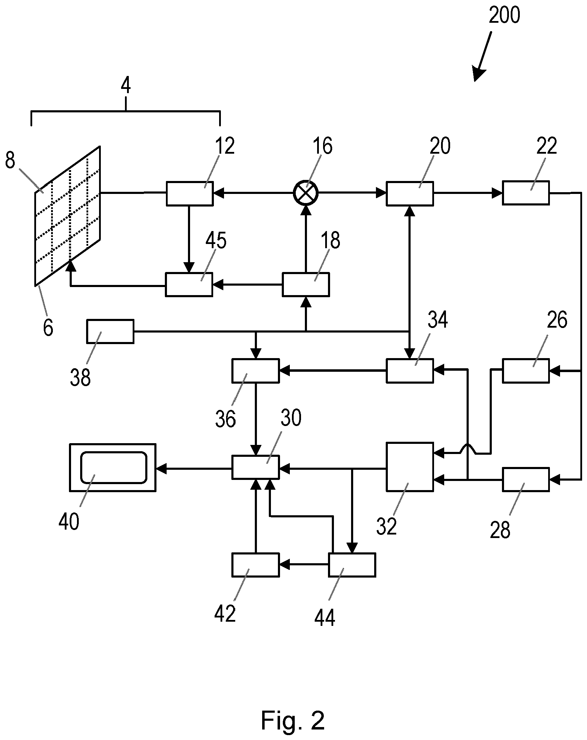

Fig. 2 shows an example ultrasound system 200. The ultrasound system 200 may comprise the apparatus 100 described above. In other embodiments, components of the ultrasound system 200 may be adapted to perform the method 1000 described below.

The system 200 comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. In this example, the transducer array 6 is a two-dimensional array of transducers 8 capable of scanning either a 2D plane or a three dimensional volume of a region of interest. In another example, the transducer array may be a 1D array.

The transducer array 6 may be coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

In an alternative embodiment, instead of a microbeamformer 12, the transducer array may be operated directly by a main system beamformer (not shown in Fig. 2).

The system 200 may further comprise a transmit/receive (T/R) switch 16, which the microbeamformer 12 can be coupled to and which switches the array between transmission and reception modes.

The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

It is noted that in an alternative embodiment, where instead of a microbeamformer 12, the transducer array is operated directly by a main system beamformer, a T/R switch 16 may protect the main beamformer 20 from high energy transmit signals.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and micro-bubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 2 only the receiver beamformers 12, 20 are shown, for simplicity. In the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 40. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image is able to depict tissue motion and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as for example,: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor may be used for making measurements in the images. The quantification processor may receive input from a user control panel 38, as described in detail below.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The graphics overlays may further contain information such as whether the transducer is monitoring in a Doppler mode or an imaging mode. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

The skilled person will appreciate that the detail provided above and the components illustrated in Fig. 2 are an example only and that an ultrasound system may have different components to those illustrated therein.

Turning now back to Fig. 1, and the functionality of the processor 102, generally, one or more transducers in an imaging mode may be used to locate (e.g. by moving the probe on the surface of the mother's stomach) the fetus. Once the fetus is located, the processor may instruct the probe to switch to a Doppler mode for subsequent monitoring of heart-rate of the located fetus. In this way, it can be ensured that it is the fetal heart that is monitored, e.g. as opposed to the maternal heart or other component of the material vascular system. This may thus facilitate improved placement of a fetal heart monitor.

In more detail, as noted above, the processor 102 is caused in step i) to obtain ultrasound image data from an ultrasound transducer.

Generally, the method may be performed in real-time, e.g. as part of a live ultrasound examination. For example, for the purposes of obstetric monitoring during labour. The processor may be caused to obtain the ultrasound image data, for example, from a probe in an imaging or B-mode of operation. The ultrasound image data may be obtained from a probe placed on the abdomen of the mother of the fetus, in order to image the uterus of the mother. Thus, in step i) the obtained ultrasound image data may comprise ultrasound image data of an uterus.

In some embodiments, a single ultrasound transducer may be used. For example, the ultrasound transducer may form part of a probe. Such a probe may be moved over the mother's abdomen (e.g. by a clinician) whilst in the imaging mode and switched to a Doppler mode when the location of the heart is located in the image data. Once in Doppler mode, the probe may be held in place, e.g. using a conventional strap or belt.

In other embodiments, the ultrasound transducer forms part of (e.g. is one of) an array of transducers. For example, an array of capacitive micromachined ultrasonic transducers, CMUTs. An array of transducers may be arranged, for example, as a wearable ultrasound patch.

The transducers in such an array may comprise, for example, capacitive micromachined ultrasonic transducers (CMUTs) arranged in a 2D matrix fashion. The CMUT sensors may share drive electronics. In this way, a large field of view ultrasound system can be formed so as to image a larger volume of the mother's abdomen, instead of trying to locate the fetal heart by moving a smaller hand held ultrasound probe over the area. The multiple CMUT sensors may be mounted on a flexible interconnect and multiplexed to the drive electronics by making use of a DC bias electrode of each device. Each CMUT array can be activated by mean of a DC voltage (also called bias voltage). As soon as a DC voltage is applied to a single CMUT, it transmits and receives ultrasound. By disconnecting the DC bias voltage from the DC bias electrode, the ultrasound sensor is left floating and it therefore does not influence the operation of the selected sensors of the set. The drive electronics are for driving and reading out the CMUT cells of the selected set.

Each ultrasound transducer may further be associated with a sensor for determining the orientation of the respective ultrasound transducer. For example, a location sensor. In some embodiments, each transducer is associated with (e.g. attached to) a respective Inertial Measurement Unit (IMU) sensor. In such embodiments, the processor may be further caused to obtain data from a plurality of IMU, sensors, each IMU being attached to a respective transducer in the array of transducers. The processor may then be caused to use the data from the IMU sensors to determine the orientation of each respective transducer. The processor may, for example, be further caused to determine the absolute position (e.g. the presentation) of the first fetus from the data from the IMU sensors, as described in more detail below.

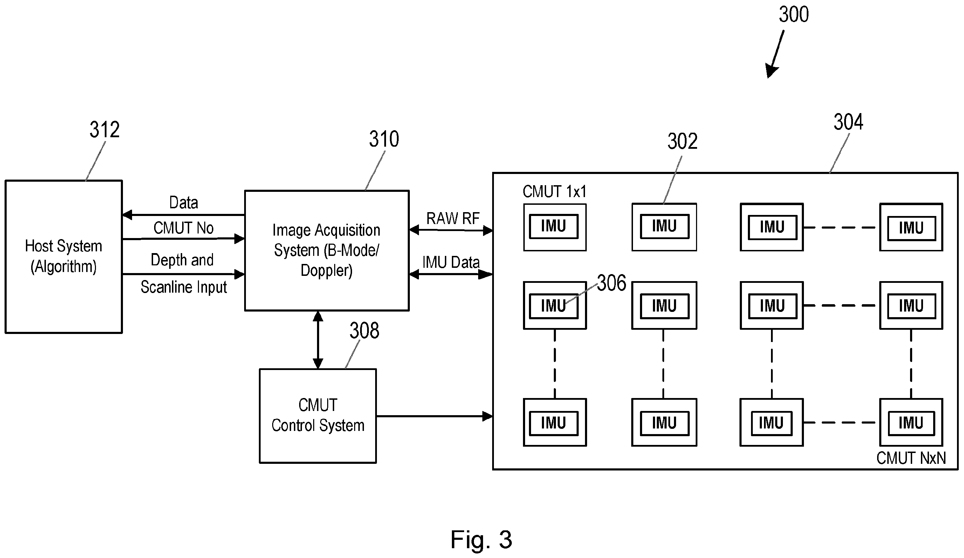

An example transducer array 300 is illustrated in Fig 3. which shows an array of transducers 302 according to an embodiment herein. In this embodiment, an *n* × *n* 2D CMUT sensors are placed on a flex PCB 302 (20mm apart). In this embodiment, on top of every CMUT sensor a 9axis IMU sensor 306 is placed. The IMU sensors provide the absolute orientation of the CMUT sensors on the flex. A CMUT control system 308 controls when each CMUT in the array is activated (for example, it may activate each CMUT in sequence, as described below). In this embodiment, the CMUT array flex is connected to an Image acquisition system 310 consisting of a micro beam former supporting B mode imaging and continuous wave Doppler mode. Raw RF data and IMU data is sent from the transducers 302 in the array to the Image acquisition system 310. Data may be analysed by a Host system 312 (such as the apparatus 100) which analyses the obtained ultrasound images from the Image acquisition system to perform steps ii) to iv) as described below.

In embodiments where the ultrasound images are acquired from an array of ultrasound transducers (e.g. such as the CMUT sensors in Fig 3) in step i) a sequence of ultrasound images may be obtained from the ultrasound transducers by activating the array of ultrasound transducers one after another in sequence.

Turning back to the processor in Fig. 1, after obtaining ultrasound image data from an ultrasound transducer, the processor is caused to perform step ii) and determine a location of the heart of a first fetus in the ultrasound image data.

In some embodiments, step ii) may be performed using machine learning. For example, the processor may be caused to determine a location of the heart of the first fetus in the ultrasound image data using a first machine learning model trained to locate fetal hearts in ultrasound images. For example, detection of the heart may be performed using deep learning techniques. For example, a neural network such as a convolutional neural network may be used. The skilled person will be familiar with machine learning models and methods of training a machine learning model to determine a location of an object in an image (e.g. via back propagation and gradient descent). The skilled person will appreciate that these are merely examples and that other types of models may also be trained to detect fetal hearts in ultrasound images.

As an example, a You Only Look Once (YOLO) based heart detection may be used to localize the heart. YOLO is described in the paper by J. Redmon and A. Farhadi. entitled "Yolov3: An incremental improvement" arXiv preprint arXiv: 1804.02767, 2018. YOLO is a region proposal network which applies a single neural network to the full image to detect certain objects. This network divides the image into regions and predicts bounding boxes and probabilities for each region. YOLO divides the input image into an S×S grid. If the centre of an object falls into a grid cell, that grid cell is responsible for detecting that object. Each grid cell predicts bounding boxes and confidence scores for those boxes. These scores reflect the reliability of the object model contained in the box and the accuracy of the box containing the predicted object. Each bounding box consists of 5 predictions: x, y, w, h, and confidence. The (x, y) coordinates represent the center of the box relative to the bounds of the grid cell. The width (w) and height (h) are predicted relative to the whole image.

In embodiments where a sequence of ultrasound images are acquired from an array of ultrasound sensors, each ultrasound image in the sequence may be analysed in turn to determine whether the image comprises an image of the fetal heart. For example, the orientation of the ultrasound (e.g. CMUT) sensors may be detected with respect to the maternal orientation using orientation sensors (such as the IMU sensors described above) that are integrated with the ultrasound transducers. Automated detection of fundal end vs cervical end of the uterus may be performed using deep learning techniques. Deep learning techniques may also be used to detect the fetal spine and fetal head. This may enable detection of the fetal presentation. Where a sequence of images is obtained, one ultrasound transducer in the array may be activated at a time, one by one in sequence across the grid. This is repeated until the heart is detected in one of the images (e.g. from one of the transducers).

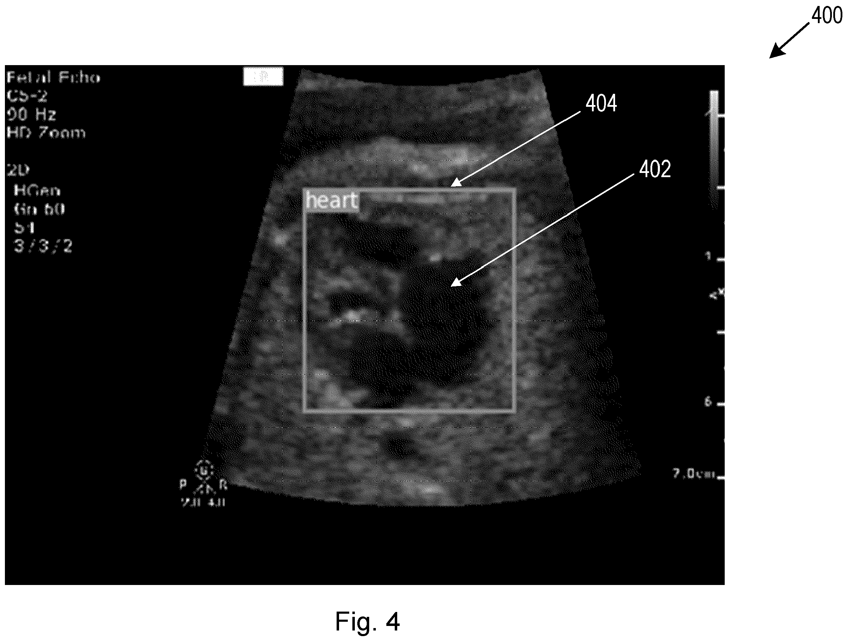

The confidence level of the YOLO output is a regression, which is trained to output the intersection of union (IOU) between output bounding box and ground truth bounding box. An example output is illustrated in Fig. 4 which shows an ultrasound image 400 of a fetal heart 402 and a bounding box 404 over the fetal heart 402 as output by a YOLO model, this image came from a single CMUT sensor.

Generally, a YOLO model may be used to determine the location of the fetal heart in an ultrasound image. For example, the depth of the heart may be obtained. In embodiments where an array of transducers is used, a YOLO model may be used to determine which transducer in the array of transducers has the fetal heart in view.

Generally, the output of step ii) may comprise, for example, an indication of the depth of the fetal heart, the scanline number and/or an indication of the ultrasound transducer where the fetal heart was detected (e.g. in examples where an array of transducers are used).

Turning back to Fig. 1, in step iii) the processor 102 may then be caused to send a message to the ultrasound transducer to instruct the ultrasound transducer to switch to a Doppler mode, wherein the determined location of the heart of the first fetus (e.g. depth and/or scanline) is used to set the target location of the Doppler mode. For example, the location (e.g. depth and/or scanline) may be used to set a Doppler gate for the Doppler mode.

In embodiments where YOLO is used, once the fetal heart has been detected, the depth, and the angle 504 at which the m-mode has to be enabled has been determined, can be passed to the imaging system.

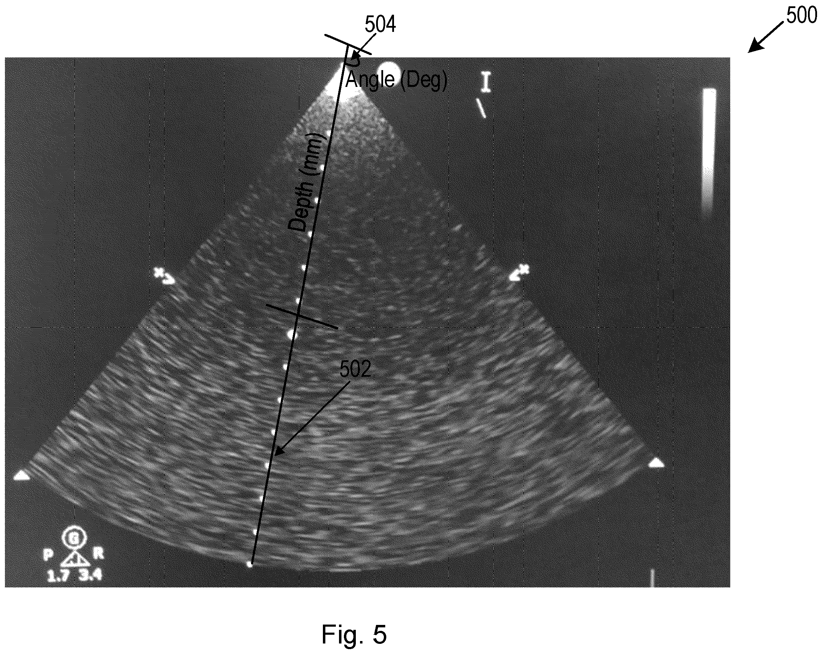

The YOLO model's output can be used to set the location for the Doppler mode e.g. the center of the bounding box predicted by the YOLO model can be used to determine the point from which the scanline passes. The angle of the scanline can also be determined. Fig. 5 shows the calculated scanline 502 from the depth information. This may be determined in a known manner. This information may then be used to set the target location of the Doppler mode (e.g. to set the Doppler gate).

In step iv) the processor is then caused to monitor the heartbeat of the first fetus using ultrasound data obtained in the Doppler mode. This may be performed in a known manner.

In embodiments as described above where in step i) ultrasound image data from each transducer in an array of transducers is obtained, the processor may be caused to select a first ultrasound transducer from the array of transducers that has a view of the heart of the first fetus; and perform steps ii), iii) and iv) with respect to the first ultrasound transducer and ultrasound image data obtained using the first ultrasound transducer.

In other words, the processor may be caused to: i) obtain ultrasound image data from an array of ultrasound transducers (e.g a sequence of ultrasound images as described above) and select a first ultrasound transducer from the array of transducers that has a view of the heart of the first fetus, ii) determine a location of the heart of the first fetus in the ultrasound image data from the first transducer iii) send a message to the first ultrasound transducer to instruct the first ultrasound transducer to switch to a Doppler mode, wherein the determined location of the heart of the first fetus is used to set the target location of the Doppler mode, and iv) monitor the heartbeat of the first fetus using ultrasound data obtained in the Doppler mode from the first transducer.

E.g. the processor causes the transducer in the array that has an image of the heart to switch to a Doppler mode in order to perform the fetal heart-monitoring. In this way an array may be used in imaging mode to determine the location of the fetal heart and then the transducer having the best view of the heart may be switched to the Doppler mode for fetal monitoring. Thus, the sonographer/clinician does not have to manually determine the optimal position of an ultrasound transducer probe - a transducer that is in the optimal position is selected in an automated manner.

As described above, movement of the fetus and/or mother may mean that over time, the placement of the ultrasound transducer needs to be updated. Or in the case of an array of transducers, a different transducer (e.g. another or second transducer) in the array may be better placed to optimally monitor the fetal heart.

Thus in some embodiments, the processor is further caused to repeat steps i)-iv) in order to re-locate the fetal heart. For example, steps i)-iv) may be performed/repeated at periodic intervals. This ensures that the location of the fetal heart is regularly monitored. In other words, the processor may cause the transducer to switch between the imaging mode + Doppler mode intermittently (e.g. every 'n' minutes) to ensure that the fetal heart (or hearts in the case of multiples as described below) is visible and audible.

In other examples, steps i)-iv) may alternatively or additionally be repeated if an audibility of the heartbeat of the first fetus falls below a threshold audibility in the ultrasound data obtained in the Doppler mode. In other words, if the quality of the heart beat signal starts to degrade, or degrades below a threshold, then this may trigger the apparatus 100 to re-locate the heart.

In other examples, steps i)-iv) may alternatively or additionally be repeated if a measure of signal to noise of the heartbeat falls below a threshold signal to noise level, and/or if the monitored heartbeat gets interrupted (or becomes ambiguous).

On switching to the imaging + Doppler mode, the apparatus re-localizes the fetal heart (e.g. using a deep learning algorithm as described above) or reconfirms the position and the depth of the fetal heart and then switches to the Doppler mode accordingly. Thus ensuring the heart is properly monitored throughout.

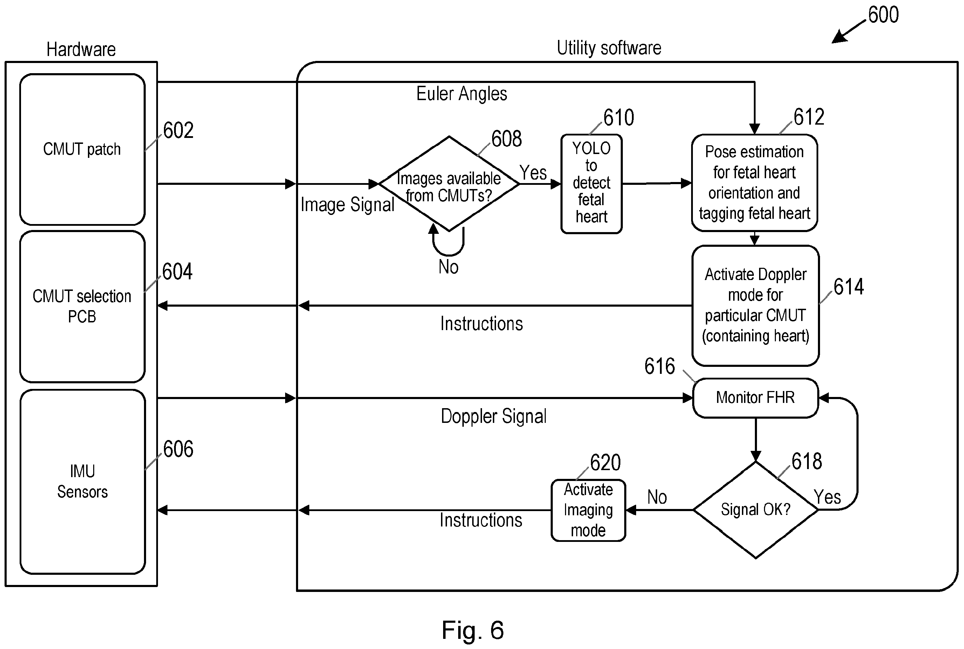

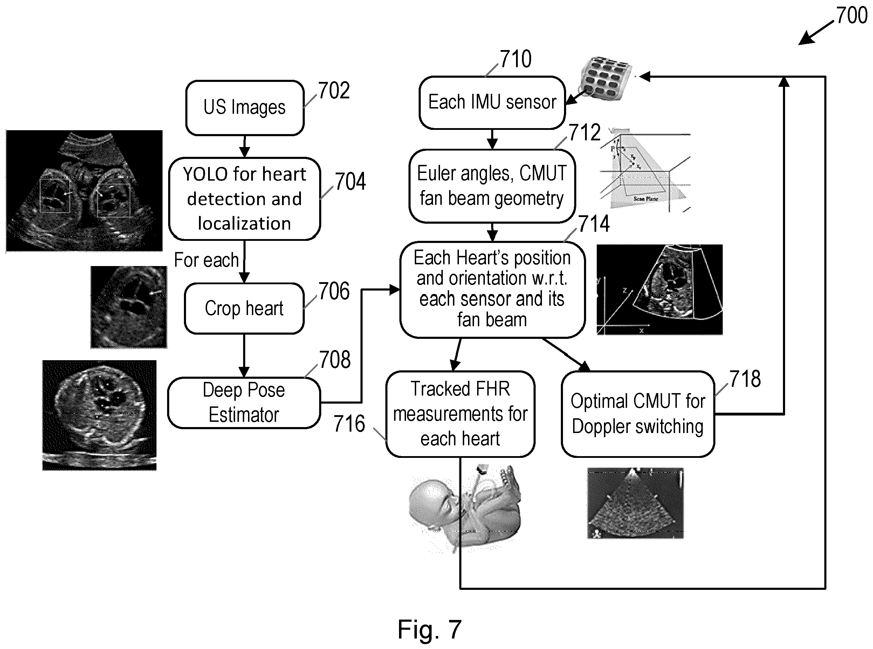

This process is illustrated in Fig. 6 which illustrates an example process 600 according to some embodiments herein. In this embodiment, there is an array of transducers mounted on a CMUT patch 602. The patch has drive-electronics in the form of a CMUT selection PCB 604 and each transducer in the array has an IMU sensor 606 attached to it. The patch provides Euler angles and image data to the apparatus 100. The apparatus 100 obtains/receives (e.g. step i) above) ultrasound image data from each transducer in the array of transducers (and may perform a check 608 that image data has been obtained) and analyses the image data from each transducer to determine a location of the heart of the first fetus in the ultrasound image data. In this embodiment illustrated in Fig. 6 a YOLO network 610 is used to identify and determine the location of the heart (as described above). Pose estimation 612 may be used to determine the orientation of the heart and/or to identify an individual heart in the case of multiple fetuses (this is described in detail below with respect to Figs 7-9). The apparatus 100 may then select a first ultrasound transducer from the array of transducers that has a view of the heart of the first fetus (as determined by the YOLO). The apparatus may then send a message 614 to the selected first ultrasound transducer to instruct the selected first ultrasound transducer 604 to switch to (e.g. activate) a Doppler mode. As noted above, the determined location of the heart of the first fetus is used to set the target location of the Doppler mode (e.g. set a Doppler gate for the Doppler mode). The location of the fetal heart may be determined at least partially using data from IMU sensors 606 on the transducers 602. In step 616, the heartbeat of the first fetus is then monitored 616 using ultrasound data obtained from the selected first transducer, in the Doppler mode. The apparatus monitors 618 the quality (e.g. signal to noise) of the Doppler signal and if the signal falls below a threshold quality, then an instruction is sent to the array of transducers to instruct the transducers to switch to an imaging mode and acquire ultrasound image data. The process is then repeated. In this way, imaging and Doppler modes may be used to efficiently and reliably monitor a fetal heart, without the need for input from a clinician.

As noted above, pregnancies involving multiples (e.g. twins, triplets etc) pose additional challenges as each fetal heart beat needs to be monitored separately. The fetal heartbeats need to be separated and reliably monitored, e.g. attributing each signal to the correct fetus and without confusing one heart beat for the other. Movements of the fetuses over time further complicate this.

In the case of a single transducer with a wide field of view, a single transducer may be used to image a second (and subsequent) fetal heart. For example, the steps ii)-iv) may be performed on two or more hearts in the same image.

For multiple pregnancies, an array of transducers as described above can enable the hearts of each fetus to be monitored in a continuous manner. For example, the processor may be further caused to select a second ultrasound transducer from the array of transducers that has a view of a heart of a second fetus, and perform steps ii), iii) and iv) with respect to the second ultrasound transducer and ultrasound image data from the second ultrasound transducer for the heartbeat of the second fetus.

In other words, the processor may be caused to: i) obtain ultrasound image data from an array of ultrasound transducers, select a first ultrasound transducer from the array of transducers that has a view of the heart of the first fetus, select a second ultrasound transducer from the array of transducers that has a view of the heart of the second fetus ii) determine a location of the heart of the first fetus in the ultrasound image data from the first transducer, determine a location of the heart of the second fetus in the ultrasound image data from the second transducer iii) send a message to the first ultrasound transducer to instruct the first ultrasound transducer to switch to a Doppler mode, wherein the determined location of the heart of the first fetus is used to set the target location of the Doppler mode of the first ultrasound transducer, send a message to the second ultrasound transducer to instruct the second ultrasound transducer to switch to a Doppler mode, wherein the determined location of the heart of the second fetus is used to set the target location of the Doppler mode of the second ultrasound transducer and iv) monitor the heartbeat of the first fetus using ultrasound data obtained in the Doppler mode from the first transducer and monitor the heartbeat of the second fetus using ultrasound data obtained in the Doppler mode from the second transducer.

The skilled person will appreciate that this process may also be generalised to third and/or subsequent fetuses.

One of the problems in current FHR monitors when monitoring multiples is that they cannot (easily) differentiate between multiple hearts i.e. which FHR is coming from which fetus. This can be further complicated because twins have a tendency to align their FHRs. In embodiments herein, landmark identification is proposed as a method for tracking each fetus to ensure that each heart beat is consistently attributed to the correct fetus. For example, each fetal heart is slightly different and thus based on the relative positions of a set of landmarks in each fetal heart, the fetal heart of the first fetus may be distinguished from the fetal heart of the second fetus in a twin pregnancy (applicable to multiples too). In this manner, key salient points may be used so that the fetuses are always tagged, even after repositioning of the transducer, and/or movements of the fetuses.

Thus in some embodiments, the processor 102 may further be caused to determine a first plurality of landmarks on the heart of the first fetus in the ultrasound image data of the first fetus and a second plurality of landmarks on the heart of the second fetus in the ultrasound image data of the second fetus. The processor may then be caused to repeat steps i)-iv) for new ultrasound image data, using the first plurality of landmarks to identify the first fetus and the second plurality of landmarks to identify the second fetus for continued monitoring of the heartbeat of the first fetus and the heartbeat of the second fetus.

Fig. 7 illustrates a method 700 of identifying, tagging and monitoring two (or more) fetal hearts using imaging and sensor information according to an example embodiment herein. In this embodiment, the fetal heart beats are monitored by combining information from a YOLO 704 (a real time object detector) followed by a real time deep learning based called Pose estimator and information from multiple IMU (inertial measurement unit) sensors. Pose estimator is described, for example, in the paper by Ke Sun, Bin Xiao, Dong Liu, and Jingdong Wang entitled: "Deep high-resolution representation learning for human pose estimation". CoRR, abs/1902.09212, 2019.

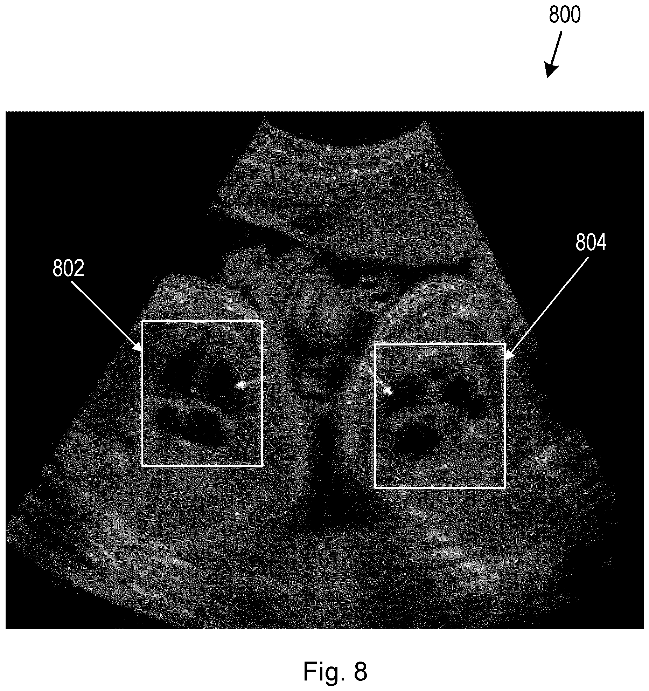

In this embodiment, YOLO 704 is run on ultrasound images 702 obtained from an array of transducers (e.g. according to step i) described above). The YOLO performs step ii) described above (e.g. the YOLO is used to determine locations of the heart of the first fetus and the second fetus in the ultrasound image data). Fig. 8 illustrates an example ultrasound image 800 and example bounding boxes 802, 804 as output by an example YOLO. With respect to Fig. 7, any bounding boxes 704 comprising detections above a certain confidence threshold, are then cropped 706 and sent independently to a pose estimator 708, to estimate the pose of each fetal heart independently. The pose estimator 708 is configured to determine a first plurality of landmarks on the heart of the first fetus in the ultrasound image data of the first fetus and a second plurality of landmarks on the heart of the second fetus in the ultrasound image data of the second fetus.

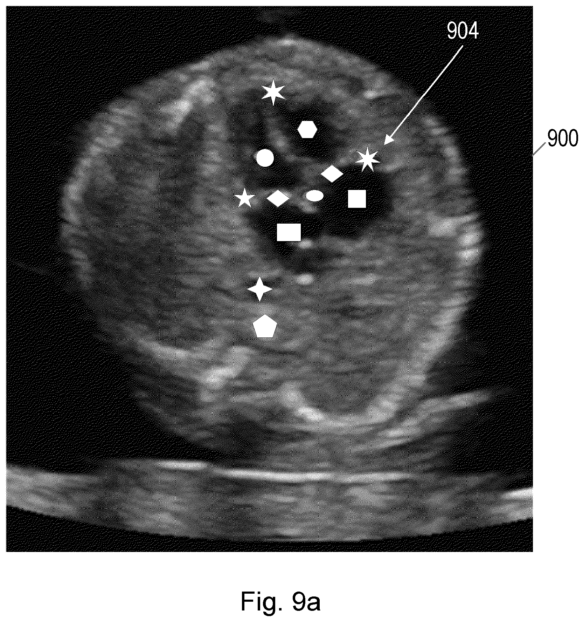

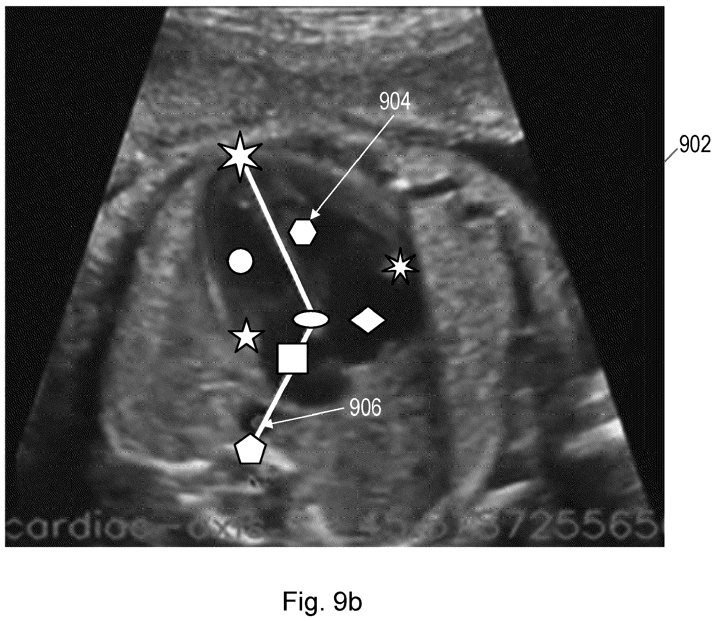

In this example, the pose identification is performed using a deep neural network which detects (e.g. regresses) landmarks of the fetal heart. An example is illustrated in Fig. 9a which shows a plurality of landmarks 904 in an image of a heart 900. Generally, landmarks may comprise, points in the cardiac anatomy and/or points on the abdominal anatomy such as the spine. Examples of landmarks include, but are not limited to, the spine, crux, tricuspid valve junction, apec, mitral valve junction, centre LV, Centre RV, Centre LA, Centre RA, Aorta, tricuspid v midpoint and the mitral v midpoint. The pose identifier may be used to track several landmarks on the heart of the first (and/or second) fetus in real-time as shown in Fig 9b. The relative orientation of the landmarks (e.g. with respect to one another) may be used to determine the orientation of the heart. As an example, and as illustrated in Fig. 9b, the angle from the spine -to- the crux of the heart -to- the apex of the heart may be used to determine the cardiac axis 906 as shown in Fig. 9b. The cardiac axis determines the absolute orientation of the fetus. The position of the crux (the position that the valves in the four chambers of the heart join) may be used as the location of the center of the heart in the Ultrasound scan.

Sensor information may be used in addition to image information. For example, in the embodiment in Fig. 7, IMU sensors 710 attached to the transducers in an array of transducers (as described above with respect to Fig. 3) may be used to determine the position and orientation of the fan beam geometry 712 of each respective transducer. Thus the orientation and position 714 of each heart w.r.t the world coordinate system can be determined. A message may be sent to the transducers in view of each fetal heart to instruct the respective ultrasound transducers to switch to a Doppler mode (using the determined location of the heart of each fetus to set the target location of the Doppler mode e.g. by setting a Doppler gate for the Doppler mode) and the heart beats of each heart may thus be monitored 716 as described above (with respect to steps iii and iv).

Using Doppler gating enables more than one transducer to be used concurrently in Doppler mode, enabling the FHRs of two or more fetuses to be monitored at the same time.

As the fetuses move, the position and orientation of each heart may be tracked by repeating the steps outlined above and using a nearest neighbour linear approximation, from the previous position and orientation.

Furthermore, subsequent acquisition may be performed in a limited FOV knowing that the lie of the baby is mostly a gradual movement (unlike limb movement or movements at an early gestational age). If at any point, the heart is not detected in the search window, the FOV may be broadened and the steps i)-iv) may be repeated for a new FOV, for example according to the "as low as reasonably achievable" (ALARA) principle. In this way, the hearts of two or more fetuses may be reliably tagged and monitored during labour, even as the fetuses move.

Turning now to Fig. 10, there is a computer implemented method of monitoring a heartbeat of a first fetus. Briefly, in a first step 1002 the method comprises i) obtaining ultrasound image data from an ultrasound transducer. In a second step 1004 the method comprises ii) determining a location of the heart of the first fetus in the ultrasound image data. In a third step 1006 the method comprises iii) sending a message to the ultrasound transducer to instruct the ultrasound transducer to switch to a Doppler mode, wherein the determined location is used to set the target location of the Doppler mode, and in a fourth step 1008 the method comprises iv) monitoring the heartbeat of the first fetus using ultrasound data obtained in the Doppler mode.

Obtaining ultrasound image data from an ultrasound transducer was described in detail above with respect to the apparatus 100 and the detail and embodiments described therein will be understood to apply equally to the method 1000.

Determining a location of the heart of the first fetus in the ultrasound image data was described in detail above with respect to the apparatus 100 and the detail and embodiments described therein will be understood to apply equally to the method 1000.

Sending a message to the ultrasound transducer to instruct the ultrasound transducer to switch to a Doppler mode, wherein the determined location is used to set the target location of the Doppler mode was also described above with respect to the apparatus 100 and the detail and embodiments described therein will be understood to apply equally to the method 1000.

Monitoring the heartbeat of the first fetus using ultrasound data obtained in the Doppler mode was also described above with respect to the apparatus 100 and the detail and embodiments described therein will be understood to apply equally to the method 1000.

In another embodiment, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus for monitoring a heartbeat of a first fetus, the apparatus comprising:
a memory comprising instruction data representing a set of instructions; and
a processor configured to communicate with the memory and to execute the set of instructions, wherein the set of instructions, when executed by the processor, cause the processor to:
obtain ultrasound image data from an ultrasound transducer;
determine a location of the heart of the first fetus in the ultrasound image data;
send a message to the ultrasound transducer to instruct the ultrasound transducer to switch to a Doppler mode, wherein the determined location of the heart of the first fetus is used to set the target location of the Doppler mode; and
monitor the heartbeat of the first fetus using ultrasound data obtained in the Doppler mode.

2. An apparatus as in claim 1 wherein the processor is caused to determine a location of the heart of the first fetus in the ultrasound image data using a first machine learning model trained to locate fetal hearts in ultrasound images.

3. An apparatus as in claim 1 or 2 wherein the processor is further caused to repeat steps i)-iv):
at periodic intervals;
if an audibility of the heartbeat of the first fetus falls below a threshold audibility in the ultrasound data obtained in the Doppler mode;
if a measure of signal to noise of the heartbeat falls below a threshold signal to noise level; and/or
if the monitored heartbeat gets interrupted.

4. An apparatus as in any one of the preceding claims wherein the ultrasound transducer forms part of an array of transducers.

5. An apparatus as in claim 4 wherein the processor is caused to:
obtain in step i) ultrasound image data from each transducer in the array of transducers;
select a first ultrasound transducer from the array of transducers that has a view of the heart of the first fetus; and
perform steps ii), iii) and iv) with respect to the first ultrasound transducer and ultrasound image data obtained using the first ultrasound transducer.

6. An apparatus as in a claim 5 wherein the processor is further caused to:
select a second ultrasound transducer from the array of transducers that has a view of a heart of a second fetus; and
perform steps ii), iii) and iv) with respect to the second ultrasound transducer and ultrasound image data from the second ultrasound transducer for the heartbeat of the second fetus.

7. An apparatus as in claim 6 wherein the processor is further caused to:
determine a first plurality of landmarks on the heart of the first fetus in the ultrasound image data of the first fetus and a second plurality of landmarks on the heart of the second fetus in the ultrasound image data of the second fetus; and
repeat steps i)-iv) for new ultrasound image data, using the first plurality of landmarks to identify the first fetus and the second plurality of landmarks to identify the second fetus for continued monitoring of the heartbeat of the first fetus and the heartbeat of the second fetus.

8. An apparatus as in claim 7 wherein the first plurality of landmarks and/or the second plurality of landmarks are determined using a second machine learning model trained to identify landmarks based on pose estimation.

9. An apparatus as in any one of claims 4 to 8 wherein the the array of transducers comprises an array of capacitive micromachined ultrasonic transducers, CMUTs.

10. An apparatus as in any one of claims 4 to 9 wherein the processor is further caused to obtain data from a plurality of inertial measurement unit, IMU, sensors, each IMU being attached to a respective transducer in the array of transducers; and
wherein the processor is caused to use the data from the IMU sensors to determine the orientation of each respective transducer.

11. An apparatus as in claim 10 wherein the processor is further caused to determine the absolute position or presentation of the first fetus from the data from the IMU sensors.

12. An ultrasound system comprising the apparatus of any one of claims 1 to 11, further comprising the ultrasound transducer.

13. An ultrasound system as in claim 12 further comprising a display, wherein the processor is caused to send an instruction to the display to display the ultrasound image data and/or the ultrasound data obtained in the Doppler mode on the display; and
wherein the processor is further caused to send an instruction to the display to cause the display to provide an indication of whether the ultrasound transducer is operating in an imaging mode or a Doppler mode.

14. A computer implemented method of monitoring a heartbeat of a first fetus, the method comprising:
obtaining ultrasound image data from an ultrasound transducer;
determining a location of the heart of the first fetus in the ultrasound image data;
sending a message to the ultrasound transducer to instruct the ultrasound transducer to switch to a Doppler mode, wherein the determined location is used to set the target location of the Doppler mode; and
monitoring the heartbeat of the first fetus using ultrasound data obtained in the Doppler mode.

15. A computer program product comprising a computer readable medium, the computer readable medium having computer readable code embodied therein, the computer readable code being configured such that, on execution by a suitable computer or processor, the computer or processor is caused to perform the method as claimed in claim 14.
